# EUROPÄISCHE PATENTANMELDUNG

(11) **EP 0 602 521 A1**
(43) Veröffentlichungstag der Anmeldung: **22.06.1994**
(21) Anmeldenummer: 93119780.0
(22) Anmeldetag: 08.12.1993
(51) Int. Cl.: C07D 471/04, A61K 31/435

(54) **Imidazopyridine**

(30) Priorität: 16.12.1992 DE 4242459
(71) Anmelder: MERCK PATENT GmbH, D-64293 Darmstadt (DE)
(72) Erfinder: Dorsch, Dieter, Dr., D-64372 Ober-Ramstadt (DE); Mederski, Werner, Dr., D-64390 Erzhausen (DE); Osswald, Mathias, Dr., D-64673 Zwingenberg (DE); Schelling, Pierre, Prof. Dr., D-64367 Mühltal (DE); Beier, Norbert, Dr., D-64354 Reinheim (DE); Lues, Ingeborg, Dr., D-64297 Darmstadt (DE); Minck, Klaus-Otto, Dr., D-64372 Ober-Ramstadt (DE)

(57) **Zusammenfassung**

Imidazopyridinderivate der Formel I 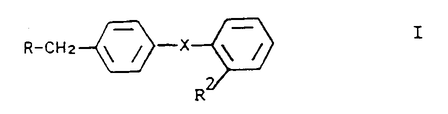
worin
- R: 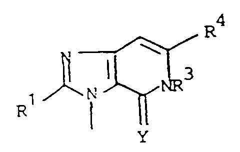
- R¹: C₃-C₇-Cycloalkyl-CₙH₂ₙ- oder C₁-C₆-Alkyl, worin eine CH₂-Gruppe durch O oder S ersetzt ist,
- R²: H, COOR⁶, CN, NO₂, NH₂, NHCOR⁵, NHSO₂R⁵ oder 1H-5-Tetrazolyl,
- R³: -CₘH₂ₘ-CN, C₂-C₆-Alkinyl, -CₘH₂ₘ-Ar, -CₘH₂ₘ-CO-R⁵, -CₘH₂ₘ-CO-Ar, -CₘH₂ₘ-Het oder -CₘH₂ₘ-CO-Het,
- R⁴: H oder Hal,
- R⁵: C₁-C₆-Alkyl, worin auch ein oder mehrere H-Atome durch F ersetzt sein können,
- R⁶: H oder A,
- X: fehlt, -NH-CO-, -CO-NH-, -O-CH(COOH)-, -NH-CH(COOH)-, -NA-CH(COOH), -CH=C(COOH), -CH=C(CN) oder -CH=C(1H-5-tetrazolyl)-,
- Y: O oder S,
- A: Alkyl mit 1-6 C-Atomen,
- Ar: eine unsubstituierte oder eine durch Hal, R⁵, OH, OR⁵, COOR⁶, CN, NO₂, NH₂, NHA, N(A)₂, NHCOR⁵, NHCOOA, NHSO₂R⁵ und/oder 1H-5-Tetrazolyl monosubstituierte oder disubstituierte Phenylgruppe,
- Het: einen fünf- oder sechsgliedrigen heteroaromatischen Rest mit 1 bis 4 N-, O- und/oder S-Atomen, der auch mit einem Benzol- oder Pyridinring kondensiert sein kann,
- Hal: F, Cl, Br oder I,
- m: 1, 2, 3, 4, 5 oder 6 und
- n: 0, 1, 2, 3, 4, 5 oder 6 bedeuten,
sowie ihre Salze,
zeigen angiotensinII-antagonistische Eigenschaften und können zur Behandlung von Hypertension, Aldosteronismus, Herzinsuffizienz und erhöhtem Augeninnendruck sowie von Störungen des Zentralnervensystems verwendet werden.

## Beschreibung

Die Erfindung betrifft neue Imidazopyridinderivate der Formel I 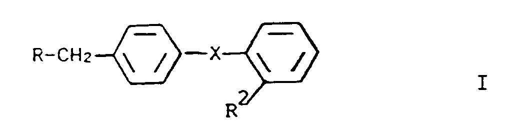
worin
- R: 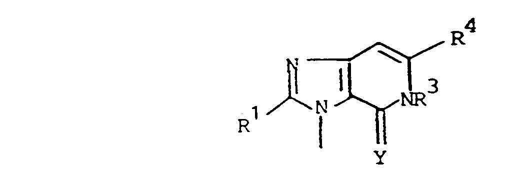
- R¹: C₃-C₇-Cycloalkyl-CₙH₂ₙ- oder C₁-C₆-Alkyl, worin eine CH₂-Gruppe durch O oder S ersetzt ist,
- R²: H, COOR⁶, CN, NO₂, NH₂, NHCOR⁵, NHSO₂R⁵ oder 1H-5-Tetrazolyl,
- R³: -CₘH₂ₘ-CN, C₂-C₆-Alkinyl, -CₘH₂ₘ-Ar, -CₘH₂ₘ-CO-R⁵, -CₘH₂ₘ-CO-Ar, -CₘH₂ₘ-Het oder -CₘH₂ₘ-CO-Het,
- R⁴: H oder Hal,
- R⁵: C₁-C₆-Alkyl, worin auch ein oder mehrere H-Atome durch F ersetzt sein können,
- R⁶: H oder A,
- X: fehlt, -NH-CO-, -CO-NH-, -O-CH(COOH)-, -NH-CH(COOH)-, -NA-CH(COOH), -CH=C(COOH), -CH=C(CN) oder -CH=C(1H-5-tetrazolyl)-,
- Y: O oder S,
- A: Alkyl mit 1-6 C-Atomen,
- Ar: eine unsubstituierte oder eine durch Hal, R⁵, OH, OR⁵, COOR⁶, CN, NO₂, NH₂, NHA, N(A)₂, NHCOR⁵, NHCOOA, NHSO₂R⁵ und/oder 1H-5-Tetrazolyl monosubstituierte oder disubstituierte Phenylgruppe,
- Het: einen fünf- oder sechsgliedrigen heteroaromatischen Rest mit 1 bis 4 N-, O- und/oder S-Atomen, der auch mit einem Benzol- oder Pyridinring kondensiert sein kann,
- Hal: F, Cl, Br oder I,
- m: 1, 2, 3, 4, 5 oder 6 und
- n: 0, 1, 2, 3, 4, 5 oder 6 bedeuten,
sowie ihre Salze.

Ähnliche Verbindungen sind aus der EP-A2-0400 974 und der EP-A1-505 893 bekannt.

Der Erfindung lag die Aufgabe zugrunde, neue Verbindungen mit wertvollen Eigenschaften aufzufinden, insbesondere solche, die zur Herstellung von Arzneimitteln verwendet werden können.

Es wurde gefunden, daß die Verbindungen der Formel I und ihre Salze bei guter Verträglichkeit sehr wertvolle pharmakologische Eigenschaften besitzen. Insbesondere zeigen sie angiotensin II-antagonistische Eigenschaften und können daher zur Behandlung der angiotensin II-abhängigen Hypertension, des Aldosteronismus, der Herzinsuffizienz und des erhöhten Augeninnendrucks sowie von Störungen des Zentralnervensystems eingesetzt werden, ferner von Hypertrophie und Hyperolasie der Blutgefäße und des Herzens, Angina pectoris, Herzinfarkt, Schlaganfall, Restenosen nach Angioplastie oder By-pass-Operationen, Arteriosklerose, Glaukomen, macularer Degeneration, Hyperurikämie, Nierenfunktionsstörungen, z.B. Nierenversagen, Nephropathia diabetica, Retinopathia diabetica, Psoriasis, angiotensin II-vermittelten Störungen in weiblichen Fortpflanzungsorganen, Wahrnehmungsstörungen, z.B. Demenz, Amnesie, Gedächtnisfunktionsstörungen, Angstzuständen, Depression und/oder Epilepsie.

Diese Wirkungen können nach üblichen in vitro- oder in vivo-Methoden ermittelt werden, wie sie z.B. in der US-PS 4 880 804, der US-PS 5 036 048 und der WO 91/14367 beschrieben sind, ferner von A.T. Chiu et al., J. Pharmacol. Exp. Therap. 250, 867-874 (1989), und von P.C. Wong et al., ibid. 252, 719-725 (1990; in vivo, an Ratten).

Die Verbindungen der Formel I können als Arzneimittelwirkstoffe in der Human- und Veterinärmedizin eingesetzt werden, insbesondere zur Prophylaxe und/oder Therapie von Herz-, Kreislauf- und Gefäßkrankheiten, vor allem von Hypertonie, Herzinsuffizienz und Hyperaldosteronismus.

Gegenstand der Erfindung sind die Verbindungen der Formel I und ihre Salze sowie ein Verfahren zur Herstellung dieser Verbindungen sowie ihrer Salze, dadurch gekennzeichnet, daß man
(a) eine Verbindung der Formel II 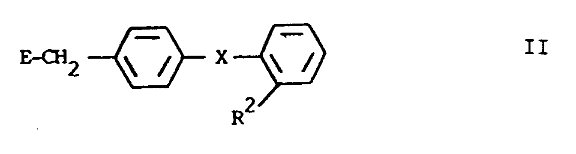 worin
   - E: Cl, Br, I oder eine freie oder reaktionsfähig funktionell äbgewandelte OH-Gruppe bedeutet und
   - R² und X: die in Anspruch 1 angegebene Bedeutung haben,
   mit einer Verbindung der Formel III

   H-R III

   worin
   - R: die in Anspruch 1 angegebene Bedeutung hat,
   umsetzt
   oder
(b) eine Verbindung der Formel IV 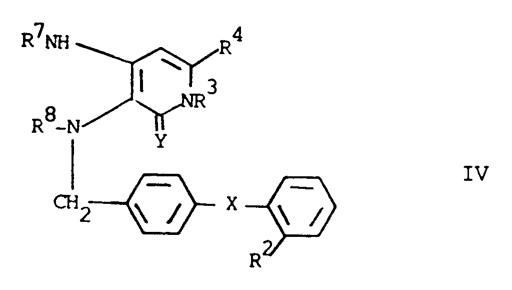 worin
   - R⁷: R¹-CO oder H und
   - R⁸: H (falls R⁷ R¹-CO ist) oder R¹-CO (falls R⁷ H ist)
   bedeuten,
   und

   - R¹, R², R³, R⁴, X und Y: die in Anspruch 1 angegebenen Bedeutungen haben,
   mit einem cyclisierenden Mittel behandelt,
   oder
(c) zur Herstellung einer Verbindung der Formel I,
   worin
   - X: -NH-CO- oder -CO-NH- bedeutet,
   eine Verbindung der Formel V 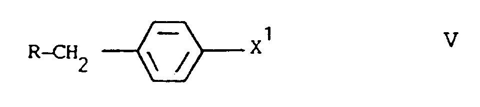 worin
   - X¹: NH₂ oder COOH bedeutet und
   - R: die in Anspruch 1 angegebene Bedeutung hat
   oder ein reaktionsfähiges Derivat dieser Verbindung mit einer Verbindung der Formel VI 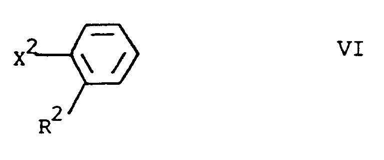 worin
   - X²: COOH (falls X¹ NH₂ ist) oder NH₂ (falls X¹ COOH ist) bedeutet und
   - R²: die in Anspruch 1 angegebene Bedeutung hat,
   oder mit einem reaktionsfähigen Derivat dieser Verbindung umsetzt,
   oder
(d) eine Verbindung der Formel I aus einem ihrer funktionellen Derivate durch Behandeln mit einem solvolysierenden oder hydrogenolysierenden Mittel in Freiheit setzt, oder
(e) eine sonst der Formel I entsprechende Verbindung, die jedoch an Stelle des Restes R³ ein H-Atom enthält, mit einer Verbindung der Formel E-R³, worin E und R³ die oben angegebenen Bedeutungen haben, behandelt,
und/oder daß man in einer Verbindung der Formel I einen oder mehrere Rest(e) R und/oder R² in einen oder mehrere andere Reste R und/oder R² umwandelt und/oder eine Base oder Säure der Formel I in eines ihrer Salze umwandelt.

Vor- und nachstehend haben die Reste bzw. Parameter R, R¹ bis R⁸, X, Y, A, Ar, Het, Hal, m, n, E, X¹ und X² die bei den Formeln I bis VI angegebenen Bedeutungen, falls nicht ausdrücklich etwas anderes angegeben ist.

In den vorstehenden Formeln hat A 1-6, vorzugsweise 1, 2, 3, oder 4 C-Atome. A bedeutet vorzugsweise Methyl, weiterhin Ethyl, Propyl, Isopropyl, Butyl, Isobutyl, sek.-Butyl, oder tert.-Butyl, ferner auch Pentyl, 1-, 2- oder 3-Methylbutyl, 1,1-, 1,2- oder 2,2-Dimethylpropyl, 1-Ethylpropyl, Hexyl, 1-, 2-, 3- oder 4-Methylpentyl, 1,1-, 1,2-, 1,3-, 2,2-, 2,3- oder 3,3-Dimethylbutyl, 1- oder 2-Ethylbutyl, 1-Ethyl-1-methylpropyl, 1-Ethyl-2-methylpropyl, 1,1,2- oder 1,2,2-Trimethylpropyl. Alkenyl steht vorzugsweise für Vinyl, 1- oder 2-Propenyl, 1-Butenyl, ferner 1-Pentenyl oder 1-Hexenyl. Alkinyl steht vorzugsweise für Ethinyl, 1- oder 2-Propinyl, ferner 1-Butinyl, 1-Pentinyl oder 1-Hexinyl.

Hal bedeutet vorzugsweise F, Cl oder Br, aber auch J.

R ist ein von 3H-Imidazo[4,5-c]pyridin ("3H-IP") abgeleiteter Rest, genauer 2-R¹-4-(thi)oxo-5-R³-6-R⁴-4,5-dihydro-3H-imidazo[4,5-c]pyridin-3-yl.

Ar ist vorzugsweise Phenyl, o-, m- oder p-Fluorphenyl, o-, m- oder p-Chlorphenyl, o-, m- oder p-Bromphenyl, o-, m- oder p-Tolyl, o-, m- oder p-Ethylphenyl, o-, m- oder p-Trifluormethylphenyl, o-, m- oder p-Hydroxyphenyl, o-, m- oder p-Methoxyphenyl, o-, m- oder p-Ethoxyphenyl, o-, m- oder p-Difluormethoxyphenyl, o-, m- oder p-Trifluormethoxyphenyl, o-, m- oder p-Carboxyphenyl, o-, m- oder p-Methoxycarbonylphenyl, o-, m- oder p-Ethoxycarbonylphenyl, o-, m- oder p-Cyanphenyl, o-, m- oder p-Nitrophenyl o-, m- oder p-Aminophenyl, o-, m- oder p-Methylaminophenyl, o-, m- oder p-Dimethylaminophenyl, o-, m- oder p-Acetamidophenyl, o-, m- oder p-Trifluoracetamidophenyl, o-, m- oder p-Methoxycarbonylaminophenyl, o-, m- oder p-Ethoxycarbonylaminophenyl, o-, m- oder p-Methylsulfonamidophenyl, o-, m- oder p-Trifluormethylsulfonamidophenyl, o-, m- oder p-(1H-5-Tetrazolyl)-phenyl, 2,3-, 2,4-, 2,5-, 2,6-, 3,4- oder 3,5-Difluorphenyl, 2,3-, 2,4-, 2,5-, 2,6-, 3,4- oder 3,5-Dichlorphenyl, 2-Fluor-4-nitro-phenyl, 2-Fluor-6-nitro-phenyl, 2-Chlor-4-nitro-phenyl, 2-Chlor-6-nitro-phenyl, 2-Ethoxycarbonyl-4-fluor-phenyl, 2-Ethoxycarbonyl-6-fluor-phenyl, 2-Chlor-4-ethoxycarbonyl-phenyl, 2-Chlor-6-ethoxycarbonyl-phenyl, 2-Fluor-4-methoxycarbonyl-phenyl, 2-Fluor-6-methoxycarbonyl-phenyl, 2-Chlor-4-methoxycarbonyl-phenyl, 2-Chlor-6-methoxycarbonyl-phenyl. Von den monosubstituierten Phenylgruppen sind die in o-Stellung substituierten bevorzugt, von den disubstituierten die in 2,6-Stellung disubstituierten.

Het ist vorzugsweise 2- oder 3-Furyl, 2- oder 3-Thienyl, 1-, 2- oder 3-Pyrrolyl, 1-, 2-, 4- oder 5-Imidazolyl, 1-, 3-, 4- oder 5-Pyrazolyl, 2-, 4- oder 5-Oxazolyl, 3-, 4- oder 5-Isoxazolyl, 2-, 4- oder 5-Thiazolyl, 3-, 4- oder 5-Isothiazolyl, 1H-1-, 1H-5-, 2H-2- oder 2H-5-Tetrazolyl, 2-, 3- oder 4-Pyridyl, 2-, 4-, 5- oder 6-Pyrimidinyl, weiterhin bevorzugt 1,2,3-Triazol-1-, -4- oder -5-yl, 1,2,4-Triazol-1-, -3- oder -5-yl, 1,2,3-Oxadiazol-4- oder -5-yl, 1,2,4-Oxadiazol-3- oder -5-yl, 1,3,4-Thiadiazol-2- oder -5-yl, 1,2,4-Thiadiazol-3 oder -5-yl, 2,1,5-Thiadiazol-3- oder -4-yl, 3- oder 4-Pyridazinyl, Pyrazinyl, 2-, 3-, 4-, 5-, 6- oder 7-Benzofuryl, 2-, 3-, 4-, 5-, 6- oder 7-Benzothienyl, 1-, 2-, 3-, 4-, 5-, 6- oder 7-Indolyl, 1-, 2-, 3-, 4-, 5-, 6- oder 7-Isoindolyl, 1-, 2-, 4- oder 5-Benzimidazolyl, 1-, 3-, 4-, 5-, 6- oder 7-Benzopyrazolyl, 2-, 4-, 5-, 6- oder 7-Benzoxazolyl, 3-, 4-, 5-, 6- oder 7-Benzisoxazolyl, 2-, 4-, 5-, 6- oder 7-Benzthiazolyl, 2-, 4-, 5-, 6- oder 7-Benzisothiazolyl, 4-, 5-, 6- oder 7-Benz-2-1,3-oxadiazolyl, 2-, 3-, 4-, 5-, 6-, 7- oder 8-Chinolyl, 1-, 3-, 4-, 5-, 6-, 7- oder 8-Isochinolyl, 3-, 4-, 5-, 6-, 7- oder 8-Cinnolyl, 2-, 4-, 5-, 6-, 7- oder 8-Chinazolyl, 1H-1-, -2-, -5-, -6- oder -7-Imidazo[4,5-b]pyridyl, 3H-2-, -3-, -5-, -6- oder -7-Imidazo[4,5-b]pyridyl, 1H-1-, -2-, -4-, -6- oder -7-Imidazo[4,5-c]pyridyl, 3H-2-, -3-, -4-, -6- oder -7-Imidazo[4,5-c]pyridyl.

In den Begriff "Het" eingeschlossen sind auch die homologen Reste, in denen der heteroaromatische Ring durch eine oder mehrere, vorzugsweise 1 oder 2, A-Grupen, vorzugsweise Methyl- und/oder Ethylgruppen substituiert ist, z.B. 3-, 4- oder 5-Methyl-2-furyl, 2-, 4- oder 5-Methyl-3-furyl, 2,4-Dimethyl-3-furyl, 3-, 4- oder 5-Methyl-2-thienyl, 3-Methyl-5-tert.-butyl-2-thienyl, 2-, 4- oder 5-Methyl-3-thienyl, 2- oder 3-Methyl-1-pyrrolyl, 1-, 3-, 4- oder 5-Methyl-2-pyrrolyl, 3,5-Dimethyl-4-ethyl-2-pyrrolyl, 2-, 4- oder 5-Methyl-1-imidazolyl, 4-Methyl-5-pyrazolyl, 4- oder 5-Methyl-3-isoxazolyl, 3- oder 5-Methyl-4-isoxazolyl, 3- oder 4-Methyl-5-isoxazolyl, 3,4-Dimethyl-5-isoxazolyl, 4- oder 5-Methyl-2-thiazolyl, 4- oder 5-Ethyl-2-thiazolyl, 2- oder 5-Methyl-4-thiazolyl, 2- oder 4-Methyl-5-thiazolyl, 2,4-Dimethyl-5-thiazolyl, 3-, 4-, 5- oder 6-Methyl-2-pyridyl, 2-, 4-, 5- oder 6-Methyl-3-pyridyl, 2- oder 3-Methyl-4-pyridyl, 4-Methyl-2-pyrimidinyl, 4,6-Dimethyl-2-pyrimidinyl, 2-, 5- oder 6-Methyl-4-pyrimidinyl, 2,6-Dimethyl-4-pyrimidinyl, 3-, 4-, 5-, 6- oder 7-Methyl-2-benzofuryl, 2-Ethyl-3-benzofuryl, 3-, 4-, 5-, 6- oder 7-Methyl-2-benzothienyl, 3-Ethyl-2-benzothienyl, 1-, 2-, 4-, 5-, 6- oder 7-Methyl-3-indolyl, 1-Methyl-5- oder 6-benzimidazolyl, 1-Ethyl-5- oder 6-benzimidazolyl.

Die Gruppen -CₘH₂ₘ- und -CₙH₂ₙ- sind vorzugsweise geradkettig und stehen somit bevorzugt für -CH₂-, ferner für -CH₂CH₂-, -(CH₂)₃-, -(CH₂)₄-, -(CH₂)₅- oder -(CH₂)₆-, aber auch z.B. für -CH(CH₃)-, -CH₂-CH(CH₃)- oder -C(CH₃)₂-. Der Parameter n kann bevorzugt auch 0 sein, so daß die Gruppe -CₙH₂ₙ- fehlt.

Der Rest R¹ ist dementsprechend vorzugsweise Cycloalkyl mit 3-7 C-Atomen, insbesondere Cyclopropyl, ferner Cyclobutyl, Cyclopentyl, Cyclohexyl, Cycloheptyl, weiterhin insbesondere Cyclopropylmethyl, 1- oder 2-Cyclopropylethyl, ferner Cyclobutylmethyl, Cyclopentylmethyl, Cyclohexylmethyl, Methoxy, Ethoxy, Propoxy, Butoxy, Isobutoxy, Methoxymethyl, Ethoxymethyl, Propoxymethyl, 2-Methoxyethyl, 3-Methoxypropyl, 2-Ethoxyethyl, Methylthio, Ethylthio, Propylthio, Butylthio, Isobutylthio, Methylthiomethyl, Ethylthiomethyl, Propylthiomethyl, 2-Methylthio-ethyl, 3-Methylthiopropyl, 2-Ethylthioethyl.

Der Rest R² ist vorzugsweise 1H-5-Tetrazolyl, ferner bevorzugt COOH, COOCH₃, COOC₂H₅, CN oder NHSO₂CF₃.

Der Rest R³ ist vorzugsweise -CH₂CN, -CH₂Ar, -CH₂-CO-R⁵, -CH₂-CO-Ar, -CH₂-Het oder -CH₂-CO-Het. Im einzelnen sind bevorzugte Bedeutungen von R⁵ Cyanalkyl (insbesondere Cyanmethyl, 2-Cyanethyl, 3-Cyanpropyl); Alkinyl (insbesondere Ethinyl, 1- oder 2-Propinyl, 1-Butin-1- oder -4-yl, 2-Butin-1-yl, 1-Pentin-1- oder -5-yl, 2-Pentin-1- oder -5-yl); unsubstituiertes oder einfach (vorzugsweise in o-Stellung) oder zweifach (vorzugsweise in 2,6-Stellung) substituiertes Aralkyl, insbesondere Benzyl, 1- oder 2-Phenylethyl, 1-, 2- oder 3-Phenylpropyl, 1-, 2-, 3- oder 4-Phenylbutyl, o-, m- oder p-Fluorbenzyl, o-, m- oder p-Chlorbenzyl, o-, m- oder p-Brombenzyl, o-, m- oder p-Methylbenzyl, o-, m- oder p-Ethylbenzyl, o-, m- oder p-Trifluormethylbenzyl, o-, m- oder p-Hydroxybenzyl, o-, m- oder p-Methoxybenzyl, o-, m- oder p-Ethoxybenzyl, o-, m- oder p-(Difluormethoxy)-benzyl, o-, m- oder p-(Trifluormethoxy)-benzyl, o-, m- oder p-Carboxybenzyl, o-, m- oder p-Methoxycarbonylbenzyl, o-, m- oder p-Ethoxycarbonylbenzyl, o-, m- oder p-Cyanbenzyl, o-, m- oder p-Nitrobenzyl, o-, m- oder p-Aminobenzyl, o-, m- oder p-Methylaminobenzyl, o-, m- oder p-Ethylaminobenzyl, o-, m- oder p-Isopropylaminobenzyl, o-, m- oder p-Dimethylaminobenzyl, o-, m- oder p-Acetamidobenzyl, o-, m- oder p-Pentanamidobenzyl, o-, m- oder p-Trifluoracetamido-benzyl, o-, m- oder p-Methoxycarbonylamino-benzyl, o-, m- oder p-tert.-Butoxycarbonylamino-benzyl, o-, m- oder p-Methylsulfonamido-benzyl, o-, m- oder p-Trifluormethylsulfonamido-benzyl, o-, m- oder p-(1H-5-Tetrazolyl)-benzyl, 2,3-, 2,4-, 2,5-, 2,6-, 3,4- oder 3,5-Difluorbenzyl, 2,3-, 2,4-, 2,5-, 2,6-, 3,4- oder 3,5-Dichlor-benzyl, 2-Chlor-6-fluor-benzyl, 2-Chlor-6-methyl-benzyl, 2-Fluor-6-trifluormethyl-benzyl, 2-Chlor-6-trifluormethyl-benzyl, 2-Fluor-6-carboxy-benzyl, 2-Fluor-6-methoxycarbonyl-benzyl, 2-Fluor-4-nitro-benzyl, 2-Fluor-6-nitro-benzyl, 2-Fluor-6-amino-benzyl, 2-Chlor-4-nitro-benzyl, 2-Chlor-6-nitro-benzyl, 2-Chlor-6-amino-benzyl, 2-Ethoxycarbonyl-4-fluorbenzyl, 2-Ethoxycarbonyl-6-fluor-benzyl, 2-Chlor-4-ethoxycarbonyl-benzyl,
2-Chlor-6-ethoxycarbonyl-benzyl, 2-Fluor-4-methoxycarbonyl-benzyl, 2-Fluor-6-methoxycarbonyl-benzyl, 2-Chlor-4-methoxycarbonyl-benzyl, 2-Chlor-6-methoxycarbonyl-benzyl, 2,3-, 2,4-, 2,5-, 2,6-, 3,4- oder 3,5-Dimethoxybenzyl; gegebenenfalls fluoriertes Oxo-alkyl, insbesondere 2-Oxopropyl, 2-Oxobutyl, 3-Methyl-2-oxobutyl, 3,3-Dimethyl-2-oxobutyl, 3,3,3-Trifluor-2-oxopropyl, 3,3,4,4,4-Pentafluor-2-oxobutyl; unsubstituiertes oder substituiertes Benzoyl-alkyl, insbesondere Phenacyl (= 2-Oxo-2-phenylethyl), o-, m- oder p-Methylphenacyl, o-, m- oder p-Ethylphenacyl, o-, m- oder p-Trifluormethylphenacyl, o-, m- oder p-Methoxyphenacyl, o-, m- oder p-Ethoxyphenacyl, o-, m- oder p-(Difluormethoxy)-phenacyl, o-, m- oder p-(Trifluormethoxy)-phenacyl, o-, m- oder p-Carboxyphenacyl, o-, m- oder p-Methoxycarbonylphenacyl, o-, m- oder p-Ethoxycarbonylphenacyl, o-, m- oder p-Cyanphenacyl, o-, m- oder p-Nitrophenacyl, o-, m- oder p-Aminophenacyl, o-, m- oder p-Acetamidophenacyl, o-, m- oder p-Trifluoracetamidophenacyl, o-, m- oder p-Methylsulfonamidophenacyl, o-, m- oder p-Trifluormethylsulfonamidophenacyl, o-, m- oder p-(1H-5-Tetrazolyl)-phenacyl; Het-alkyl, insbesondere 2- oder 3-Furylmethyl, 2- oder 3-Thienylmethyl, 5-Isoxazolylmethyl, 5-Methyl-3-isoxazolylmethyl, 2-, 3- oder 4- Pyridylmethyl, Pyrazinylmethyl, 2-, 4-, 5- oder 6-Pyrimidinylmethyl, 3- oder 4-Pyridazinylmethyl, 2-, 3-, 4-, 5-, 6- oder 7-Benzofurylmethyl, 2-, 3-, 4-, 5-, 6- oder 7-Benzothienylmethyl, 2-, 3-, 4-, 5-, 6- oder 7-Indolylmethyl; Het-CO-alkyl, insbesondere 2-Furoylmethyl, 2-Thenoylmethyl, Picolinoylmethyl, Nicotinoylmethyl, Isonicotinoylmethyl, Pyrazin-carbonylmethyl, 2-, 4-, 5- oder 6-Pyrimidincarbonylmethyl, 3- oder 4-Pyridazincarbonylmethyl, Benzofuran-2-, -3-, -4-, -5-, -6- oder -7-carbonylmethyl, Benzothiophen-2-, -3-, -4-, -5-, -6- oder -7-carbonylmethyl, Indol-2-, -3-, -4-, -5-, -6- oder -7-carbonylmethyl. Von den substituierten Phenacylgruppen sind die in p-Stellung substituierten bevorzugt.

Der Rest R⁴ ist bevorzugt H, aber auch F, Cl, Br oder I.

Der Rest R⁵ enthält bevorzugt 1, 2 oder 3-C-Atome und bedeutet vorzugsweise Methyl, Ethyl, Trifluormethyl, Pentafluroethyl, 2,2,2-Trifluorethyl oder 3,3,3-Trifluorpropyl. Falls eine Verbindung der Formel I zwei Reste R⁵ enthält, so können sie gleich oder voneinander verschieden sein.

Der Rest R⁶ ist vorzugsweise H, ferner bevorzugt Methyl oder Ethyl.

Der Rest X fehlt vorzugsweise oder bedeutet vorzugsweise -NH-CO- oder -CO-NH-.

Der Rest Y ist vorzugsweise O, aber auch S.

Die Verbindungen der Formel I können ein oder mehrere chirale Zentren besitzen und daher in verschiedenen - optisch-aktiven oder optisch-inaktiven - Formen vorkommen. Die Formel I umschließt alle diese Formen.

Dementsprechend sind Gegenstand der Erfindung insbesondere diejenigen Verbindungen der Formel I, in denen mindestens einer der genannten Reste eine der vorstehend angegebenen bevorzugten Bedeutungen hat. Einige bevorzugte Gruppen von Verbindungen können durch die folgenden Teilformeln Ia bis Ii ausgedrückt werden, die der Formel I entsprechen und worin die nicht näher bezeichneten Reste die bei Formel I angegebenen Bedeutungen haben, worin jedoch:
in Ia X fehlt;
in Ib X -NH-CO- bedeutet;
in Ic X -CO-NH- bedeutet;
in Id X -O-CH(COOH)- bedeutet;
in Ie X -NH―CH(COOH)- bedeutet;
in If X -NA-CH(COOH)- bedeutet;
in Ig X -CH=C(COOH)- bedeutet;
in Ih X -CH=C(CN)- bedeutet;
in Ii X -CH=C(1H-5-tetrazolyl)- bedeutet.

Verbindungen der Formel Ia sind besonders bevorzugt.

Weiterhin sind bevorzugt:
Verbindungen der Formeln Ik sowie Iak bis Iik, die den Verbindungen der Formeln I sowie Ia bis Ii entsprechen, worin jedoch zusätzlich Y ein O-Atom bedeutet;
Verbindungen der Formeln Il, Ial bis Ikl, sowie Iakl bis Iikl, die den Formeln I, Ia bis Ik sowie Iak bis Iik entsprechen, worin jedoch zusätzlich R⁴ H bedeutet;
Verbindungen der Formeln Im, Iam bis Ilm, Ialm bis Iklm sowie Iaklm bis Iiklm, die den Formeln I, Ia bis Il, Ial bis Ikl sowie Iakl bis Iikl entsprechen, worin jedoch zusätzlich R² CN oder 1H-5-Tetrazolyl bedeutet.

Unter diesen sind diejenigen Verbindungen bevorzugt, worin R¹ Cyclopropyl bedeutet.

Weitere bevorzugte Gruppen von Verbindungen entsprechen der Formel I sowie den anderen vorstehend genannten Formeln, worin jedoch der Rest R³
(a) R⁵-CO-CH₂-,
(b) Ar-CO-CH₂-,
(c) Het-CO-CH₂-,
(d) Het-CH₂-
(e) p-Aminophenacyl oder
(f) o-COOR⁶-benzyl bedeutet.

Eine kleine ausgewählte Gruppe bevorzugter Verbindungen entspricht der Formel I, worin
- R: einen 2-Cyclopropyl-4,5-dihydro-4-oxo-5-R³-3H-imidazo[4,5-c]pyridin-3-yl-rest,
- R²: 1H-5-Tetrazolyl und
- R³: o-Methoxycarbonylbenzyl oder o-Ethoxycarbonylbenzyl bedeutet
und
- X: fehlt.

Die Verbindungen der Formel I und auch die Ausgangsstoffe zu ihrer Herstellung werden im übrigen nach an sich bekannten Methoden hergestellt, wie sie in der Literatur (z.B. in den Standardwerken wie Houben-Weyl, Methoden der organischen Chemie, Georg-Thieme-Verlag, Stuttgart; insbesondere aber in der EP-A2-0 430 709 und in der US-PS 4 880 804) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Die Ausgangsstoffe können, falls erwünscht, auch in situ gebildet werden, so daß man sie aus dem Reaktionsgemisch nicht isoliert, sondern sofort weiter zu den Verbindungen der Formel I umsetzt.

Die Verbindungen der Formel I können vorzugsweise erhalten werden, indem man Verbindungen der Formel II mit Verbindungen der Formel III umsetzt. Besonders die Biphenylderivate der Formel I (worin X fehlt) sind auf diesem Wege gut erhältlich.

In den Verbindungen der Formel II bedeutet E vorzugsweise Cl, Br, I oder eine reaktionsfähig funktionell abgewandelte OH-Gruppe wie Alkylsulfonyloxy mit 1-6 C-Atomen (bevorzugt Methylsulfonyloxy) oder Arylsulfonyloxy mit 6-10 C-Atomen (bevorzugt Phenyl- oder p-Tolylsulfonyloxy).

Die Umsetzung von II mit III erfolgt zweckmäßig, indem man zunächst III durch Behandeln mit einer Base in ein Salz umwandelt, z.B. mit einem Alkalimetallalkoholat wie CH₃ONa oder K-tert.-Butylat in einem Alkohol wie CH₃OH oder mit einem Alkalimetallhydrid wie NaH oder mit einem Alkalimetallalkoholat in Dimethylformamid (DMF), und dieses dann in einem inerten Lösungsmittel, z.B. einem Amid wie DMF, N-Methylpyrrolidinon oder Dimethylacetamid oder einem Sulfoxid wie

Dimethylsulfoxid (DMSO), mit II umsetzt, zweckmäßig bei Temperaturen zwischen -20 und 100°, vorzugsweise zwischen 10 und 30°. Als Basen eignen sich auch Alkalimetallcarbonate wie Na₂CO₃ oder K₂CO₃ oder Alkalimetall-hydrogencarbonate wie NaHCO₃ oder KHCO₃.

Die Verbindungen der Formel I sind weiterhin durch Cyclisierung von Verbindungen der Formel IV erhältlich. Diese Cyclisierung gelingt zweckmäßig durch Erhitzen mit Polyphosphorsäure, Essigsäure oder Diglyme auf Temperaturen zwischen etwa 80 und 180°, vorzugsweise zwischen 120 und 160°.

Säureamide der Formel I (X = -NH-CO- oder -CO-NH-) sind ferner erhältlich durch Umsetzung von Verbindungen der Formel V (oder ihrer reaktionsfähigen Derivate) mit Verbindungen der Formel VI (oder ihren reaktionsfähigen Derivaten).

Als reaktionsfähige Derivate der Carbonsäuren der Formeln V und VI (X¹ bzw. X² = COOH) eignen sich vorteilhaft die entsprechenden Chloride, Bromide oder Anhydride. Die Umsetzung erfolgt zweckmäßig in Gegenwart eines inerten Lösungsmittels, z.B. eines halogenierten Kohlenwasserstoffs wie Dichlormethan, Chloroform, Trichlorethen oder 1,2-Dichlorethan oder eines Ethers wie Tetrahydrofuran (THF) oder Dioxan, bei Temperaturen zwischen 0 und 150°, vorzugsweise zwischen 20 und 80°. Setzt man Säurehalogenide um, so empfiehlt sich der Zusatz einer Base, z.B. eines tertiären Amins wie Triethylamin, Pyridin oder 4-Dimethylaminopyridin.

Man kann ferner eine Verbindung der Formel I aus einem ihrer funktionellen Derivate durch Behandeln mit einem solvolysierenden (z.B. hydrolysierenden) oder hydrogenolysierenden Mittel in Freiheit setzen.

So ist es möglich, nach einer der angegebenen Methoden eine Verbindung herzustellen, die der Formel I entspricht, aber an Stelle einer 5-Tetrazolylgruppe eine in 1- oder 2-Stellung funktionell abgewandelte (durch eine Schutzgruppe geschützte) 1H- oder 2H-5-Tetrazolylgruppe enthält. Als Schutzgruppen eignen sich beispielsweise: Triphenylmethyl, abspaltbar mit HCl oder Ameisensäure in einem inerten Lösungsmittel oder Lösungsmittelgemisch, z.B. Ether/Dichlormethan/Methanol; 2-Cyanethyl, abspaltbar mit NaOH in Wasser/THF; p-Nitrobenzyl, abspaltbar mit H₂/Raney-Nickel in Ethanol.

Ferner kann man Carbonsäuren der Formel I, worin X -O-CH(COOH), -NH-CH(COOH), -NA-CH(COOH) oder -CH=C(COOH) bedeutet, erhalten durch Verseifung entsprechender Alkylester, z.B. mit NaOH oder KOH in wässeriger Lösung mit oder ohne Zusatz eines inerten organischen Lösungsmittels wie Methanol, Ethanol, THF oder Dioxan bei Temperaturen zwischen 0 und 100°, oder durch Hydrogenolyse entsprechender Benzylester, z.B. an Pd-Kohle bei Drucken zwischen 1 und 200 bar und bei Temperaturen zwischen 0 und 100° in einem der angegebenen inerten Lösungsmittel.

Weiterhin kann man eine sonst der Formel I entsprechende Verbindung, die jedoch an Stelle des Restes R³ ein H-Atom enthält, mit einer Verbindung der Formel E-R³ behandeln.

Typische Verbindungen der Formel E-R³ sind z.B. Chlor- oder Bromacetonitril, Propargylchlorid oder -bromid, Benzylchlorid oder -bromid, o-Chlormethyl- oder o-Brommethyl-benzoesäuremethyl- oder - ethylester, Chlor- oder Bromaceton, Phenacylchlorid oder -bromid, 2-Thienylmethylchlorid oder -bromid, 2-Furoylmethylchlorid oder -bromid.

Man arbeitet bevorzugt in einem Säureamid wie DMF, N-Methylpyrrolidon, 1,3-Dimethyl-2-oxo-hexahydropyrimidin oder Phosphorsäure-hexamethyl-triamid, einem Alkohol wie Methanol oder tert.-Butanol, einem Ether wie THF oder einem halogenierten Kohlenwasserstoff wie Dichlormethan oder Gemischen davon als Lösungsmittel und/oder in Gegenwart eines Alkalimetallalkoholats wie Natriummethylat oder Kalium-tert.-butylat, eines Alkalimetallhydrids wie Natrium- oder Kaliumhydrid, eines Alkalimetallcarbonats wie Natrium- oder Kaliumcarbonat, eines Alkalimetallbicarbonats wie Natrium- oder Kaliumbicarbonat oder eines tertiären Amins wie Triethylamin oder Ethyldiisopropylamin bei Temperaturen zwischen etwa -30 und 200, vorzugsweise zwischen 20 und 60°.

Die Ausgangsstoffe, insbesondere diejenigen der Formel II sind teilweise bekannt. Falls sie nicht bekannt sind, können sie nach bekannten Methoden in Analogie zu bekannten Stoffen hergestellt werden. Verbindungen der Formel III (R⁴ = H, Y = O) sind z.B. erhältlich durch Reaktion von Carbonsäuren der Formel R¹-COOH mit Verbindungen der Formel VII 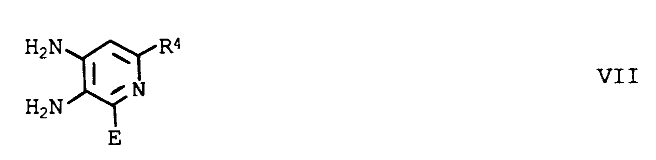
in Gegenwart von Polyphosphorsäure; dabei wird die Gruppe E (vorzugsweise Cl) hydrolysiert, und es entstehen zunächst Verbindungen entsprechend III, worin jedoch an Stelle von R³ ein H-Atom steht; diese werden anschließend mit Verbindungen der Formel E-R³ umgesetzt.

Verbindungen der Formel V sind z.B. erhältlich durch Umsetzung von Verbindungen der Formel VIII 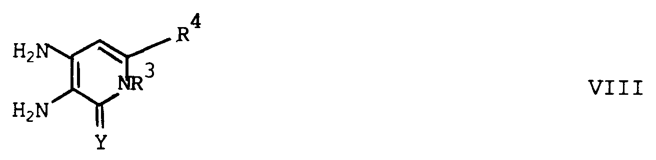
worin jedoch die eine Aminogruppe durch eine Aminoschutzgruppe (z.B. Benzyl, A-O-CO- oder Benzyloxycarbonyl) geschützt ist, mit Verbindungen der Formel II und nachfolgende Abspaltung der Schutzgruppe und Reaktion mit Säuren der Formel R¹-COOH oder deren funktionellen Derivaten; sie werden in der Regel nicht isoliert, sondern entstehen in situ bei der letztgenannten Umsetzung.

Es ist ferner möglich, eine Verbindung der Formel I in eine andere Verbindung der Formel I umzuwandeln, indem man einen oder mehrere der Reste R und/oder R² in andere Reste R und/oder R² umwandelt, z.B. indem man Nitrogruppen (z.B. durch Hydrierung an Raney-Nickel oder Pd-Kohle in einem inerten Lösungsmittel wie Methanol oder Ethanol) zu Aminogruppen reduziert und/oder freie Amino- und/oder Hydroxygruppen funktionell abwandelt und/oder funktionell abgewandelte Amino- und/oder Hydroxygruppen durch Solvolyse oder Hydrogenolyse freisetzt und/oder Nitrilgruppen zu COOH-Gruppen hydrolysiert oder mit Derivaten der Stickstoffwasserstoffsäure, z.B. Natriumazid in N-Methylpyrrolidon oder Trimethylzinnazid in Toluol, zu Tetrazolylgruppen umsetzt.

So kann man beispielsweise freie Aminogruppen in üblicher Weise mit einem Säurechlorid oder -anhydrid acylieren oder mit einem unsubstituierten oder substituierten Alkyl-halogenid alkylieren, zweckmäßig in einem inerten Lösungsmittel wie Dichlormethan oder THF und/oder in Gegenwart einer Base wie Triethylamin oder Pyridin bei Temperaturen zwischen -60 und +30°.

Gewünschtenfalls kann in einer Verbindung der Formel I eine funktionell abgewandelte Amino- und/oder Hydroxygruppe durch Solvolyse oder Hydrogenolyse nach üblichen Methoden in Freiheit gesetzt werden. So kann z.B. eine Verbindung der Formel I, die eine NHCOR⁵- oder eine COOA-Gruppe enthält, in die entsprechende Verbindung der Formel I umgewandelt werden, die stattdessen eine NH₂- oder eine COOH-Gruppe enthält. COOA-Gruppen können z.B. mit NaOH oder KOH in Wasser, Wasser-THF oder Wasser-Dioxan bei Temperaturen zwischen 0 und 100° verseift werden.

Umsetzung von Nitrilen der Formel I (z.B. solche mit R² = CN) mit Derivaten der Stickstoffwasserstoffsäure führt zu Tetrazolen der Formel I (z.B. mit R² = 1H-5-Tetrazolyl). Bevorzugt verwendet man Trialkylzinnazide wie Trimethylzinnazid in einem inerten Lösungsmittel, z.B. einem aromatischen Kohlenwasserstoff wie Toluol bei Temperaturen zwischen 20 und 150°, vorzugsweise zwischen 80 und 140°, oder Natriumazid in N-Methylpyrrolidon bei Temperaturen zwischen etwa 100 und 200°.

Eine Base der Formel I kann mit einer Säure in das zugehörige Säureadditionssalz übergeführt werden. Für diese Umsetzung kommen insbesondere Säuren in Frage, die physiologisch unbedenkliche Salze liefern, So können anorganische Säuren verwendet werden, z.B. Schwefelsäure, Salpetersäure, Halogenwasserstoffsäuren wie Chlorwasserstoffsäure oder Bromwasserstoffsäure, Phosphorsäuren wie Orthophosphorsäure, Sulfaminsäure, ferner organische Säuren, insbesondere aliphatische, alicyclische, araliphatische, aromatische oder heterocyclische ein- oder mehrbasige Carbon-, Sulfon- oder Schwefelsäuren, z.B. Ameisensäure, Essigsäure, Propionsäure, Pivalinsäure, Diethylessigsäure, Malonsäure, Bernsteinsäure, Pimelinsäure, Fumarsäure, Maleinsäure, Milchsäure, Weinsäure, Äpfelsäure, Citronensäure, Gluconsäure, Ascorbinsäure, Nicotinsäure, Isonicotinsäure, Methan- oder Ethansulfonsäure, Ethandisulfonsäure, 2-Hydroxyethansulfonsäure, Benzolsulfonsäure, p-Toluolsulfonsäure, Naphthalin-mono-und -disulfonsäuren, Laurylschwefelsäure. Salze mit physiologisch nicht unbedenklichen Säuren, z.B. Pikrate, können zur Isolierung und/oder Aufreinigung der Verbindungen der Formel I verwendet werden.

Andererseits können Verbindungen der Formel I, die COOH- oder Tetrazolylgruppen enthalten, mit Basen (z.B. Natrium- oder Kaliumhydroxid oder -carbonat) in die entsprechenden Metall-, insbesondere Alkalimetall- oder Erdalkalimetall-, oder in die entsprechenden Ammoniumsalze umgewandelt werden. Die Kaliumsalze der Tetrazolylderivate sind besonders bevorzugt.

Die neuen Verbindungen der Formel I und ihre physiologisch unbedenklichen Salze können zur Herstellung pharmazeutischer Präparate verwendet werden, indem man sie zusammen mit mindestens einem Träger- oder Hilfsstoff und, falls erwünscht, zusammen mit einem oder mehreren weiteren Wirkstoff(en) in eine geeignete Dosierungsform bringt. Die so erhaltenen Zubereitungen können als Arzneimittel in der Human- oder Veterinärmedizin eingesetzt werden. Als Trägersubstanzen kommen organische oder anorganische Stoffe in Frage, die sich für die enterale (z.B. orale oder rektale) oder parenterale Applikation oder für eine Applikation in Form eines Inhalations-Sprays eignen und mit den neuen Verbindungen nicht reagieren, beispielsweise Wasser, pflanzliche Öle, Benzylalkohole, Polyethylenglykole, Glycerintriacetat und andere Fettsäureglyceride, Gelatine, Sojalecithin, Kohlehydrate wie Lactose oder Stärke, Magnesiumstearat, Talk, Cellulose. Zur oralen Anwendung dienen insbesondere Tabletten, Dragees, Kapseln, Sirupe, Säfte oder Tropfen; von Interesse sind speziell Lacktabletten und Kapseln mit magensaftresistenten Überzügen bzw. Kapselhüllen. Zur rektalen Anwendung dienen Suppositorien, zur parenteralen Applikation Lösungen, vorzugsweise ölige oder wässerige Lösungen, ferner Suspensionen, Emulsionen oder Implantate. Für die Applikation als Inhalations-Spray können Sprays verwendet werden, die den Wirkstoff entweder gelöst oder suspendiert in einem Treibgasgemisch (z.B. Fluorchlorkohlenwasserstoffen) enthalten. Zweckmäßig verwendet man den Wirkstoff dabei in mikronisierter Form, wobei ein oder mehrere zusätzliche physiologisch verträgliche Lösungsmittel zugegen sein können, z.B. Ethanol. Inhalationslösungen können mit Hilfe üblicher Inhalatoren verabfolgt werden. Die neuen Verbindungen können auch lyophilisiert und die erhaltenen Lyophilisate z.B. zur Herstellung von Injektionspräparaten verwendet werden. Die angegebenen Zubereitungen können sterilisiert sein und/oder Hilfsstoffe wie Konservierungs-, Stabilisierungs- und/oder Netzmittel, Emulgatoren, Salze zur Beeinflussung des osmotischen Druckes, Puffersubstanzen, Farb- und/oder Aromastoffe enthalten. Sie können, falls erwünscht, auch einen oder mehrere weitere Wirkstoffe enthalten, z.B. ein oder mehrere Vitamine, Diuretika, Antiphlogistika.

Die erfindungsgemäßen Substanzen werden in der Regel in Analogie zu anderen bekannten, im Handel befindlichen Präparaten, insbesondere aber in Analogie zu den in der US-PS 4 880 804 beschriebenen Verbindungen verabreicht, vorzugsweise in Dosierungen zwischen etwa 1 mg und 1 g, insbesondere zwischen 50 und 500 mg pro Dosierungseinheit. Die tägliche Dosierung liegt vorzugsweise zwischen etwa 0,1 und 50 mg/kg, insbesondere 1 und 10 mg/kg Körpergewicht. Die spezielle Dosis für jeden bestimmten Patienten hängt jedoch von den verschiedensten Faktoren ab, beispielsweise von der Wirksamkeit der eingesetzten speziellen Verbindung, vom Alter, Körpergewicht, allgemeinen Gesundheitszustand, Geschlecht, von der Kost, vom Verabfolgungszeitpunkt und -weg, von der Ausscheidungsgeschwindigkeit, Arzneistoffkombination und Schwere der jeweiligen Erkrankung, welcher die Therapie gilt. Die orale Applikation ist bevorzugt.

Vor- und nachstehend sind alle Temperaturen in °C angegeben. In den nachfolgenden Beispielen bedeutet "übliche Aufarbeitung": Man gibt, falls erforderlich, Wasser hinzu, stellt, falls erforderlich, je nach Konstitution des Endprodukts auf pH-Werte zwischen 2 und 10 ein, extrahiert mit Ethylacetat oder Dichlormethan, trennt ab, trocknet die organische Phase über Natriumsulfat, dampft ein und reinigt durch Chromatographie an Kieselgel und/oder durch Kristallisation. IP = Imidazo[4,5-c]pyridin. Rf-Werte an Kieselgel (dünnschichtchromatographisch), FAB = Massenspektrum, erhalten nach der "fast atom bombardment"-Methode, (M+H)⁺-Peak.

### Beispiel 1

(a) Eine Lösung von 0,23 g Na in 20 ml Methanol wird innerhalb 15 Minuten zugetropft zu einer Lösung von 2,55 g 2-Cyclopropyl-5-(2-furylmethyl)-4,5-dihydro-4-oxo-3H-IP [erhältlich durch Kondensation von Cyclopropancarbonsäure mit 3,4-Diamino-2-chlorpyridin in Gegenwart von Polyphosphorsäure zu 2-Cyclopropyl-4,5-dihydro-4-oxo-1(oder 3)H-IP, Reaktion mit Benzylbromid in Methanol in Gegenwart von CH₃ONa zu 3-Benzyl-2-cyclopropyl-4,5-dihydro-4-oxo-3H-IP, Reaktion mit 2-Furylmethylchlorid in DMF in Gegenwart von K-tert.-Butylat zu 3-Benzyl-2-cyclopropyl-5-(2-furylmethyl)-4,5-dihydro-4-oxo-3H-IP und hydrogenolytische Abspaltung der Benzylgruppe] in 75 ml Methanol. Man rührt noch 30 Minuten bei 20°, dampft ein, löst den Rückstand in 20 ml DMF und tropft bei 0° unter Rühren eine Lösung von 3,05 g 4'-Brommethyl-biphenyl-2-carbonsäure-methylester (IIa) in 10ml DMF hinzu. Man rührt 16 Stunden bei 20°, dampft ein, arbeitet wie üblich auf, chromatographiert an Kieselgel und erhält 2-Cyclopropyl-5-(2-furylmethyl)-4,5-dihydro-3-(2'-methoxycarbonyl-biphenylyl-4-methyl)-4-oxo-3H-IP.
(b) Ein Gemisch von 1 g des nach (a) erhaltenen Methylesters, 12 ml wässeriger 2 n NaOH-Lösung und 48 ml Methanol wird 2 Std. gekocht, dann eingedampft. Man arbeitet wie üblich auf (wässerige Salzsäure bis pH 3/Dichlormethan) und erhält 2-Cyclopropyl-5-(2-furylmethyl)-4,5-dihydro-3-(2'-carboxy-biphenylyl-4-methyl)-4-oxo-3H-IP.

### Beispiel 2

Analog Beispiel 1 erhält man aus 3,37 g 2-Cyclopropyl-5-(o-ethoxycarbonylbenzyl)-4,5-dihydro-4-oxo-3H-IP und 2,98 g 3-p-Brommethyl-phenyl-2-phenyl-acrylnitril [F. 178°; erhältlich durch Kondensation von p-Tolylaldehyd mit Phenylacetonitril in Gegenwart von C₂H₅ONa in Ethanol zu 2-Phenyl-3-p-tolyl-acrylnitril (F. 61°) und Bromierung mit N-Bromsuccinimid in Dichlormethan] das 2-Cyclopropyl-5-(o-ethoxycarbonylbenzyl)-3-[p-(2-cyan-2-phenyl-vinyl)-benzyl]-4,5-dihydro-4-oxo-3H-IP.

### Beispiel 3

Ein Gemisch von 0,86 g Cyclopropancarbonsäure, 4,55 g 4-Amino-1,2-dihydro-2-oxo-3-[2'-(1H-5-tetrazolyl)-biphenylyl-4-methyl-amino-1-(o-ethoxycarbonylbenzyl)-pyridin [erhältlich durch Reaktion von 3-Amino-4-benzylamino-1,2-dihydro-2-oxo-1-(o-ethoxycarbonylbenzyl)-pyridin mit 4-Brommethyl-2'-cyan-biphenyl zu 4-Benzylamino-3-(2'-cyan-biphenylyl-4-methyl-amino)-1,2-dihydro-2-oxo-1-(o-ethoxycarbonylbenzyl)-pyridin, Reaktion mit Trimethylzinnazid zu 4-Benzylamino-1,2-dihydro-2-oxo-3-[2'-(1H-5-tetrazolyl)-biphenylyl-4-methyl-amino]-1-(o-ethoxycarbonylbenzyl)-pyridin und hydrogenolytische Abspaltung der Benzylgruppe] und 50 g Polyphosphorsäure wird 5 Stunden auf 140° erhitzt. Als Zwischenprodukte entstehend in situ 4-Amino-1,2-dihydro-2-oxo-3-[N-(2'-(1H-5-tetrazolyl)-biphenylyl]-4-methyl-N-cyclopropylcarbonylamino]-1-(o-ethoxycarbonylbenzyl)-pyridin und 1,2-Dihydro-2-oxo-3-[2'-(1H-5-tetrazolyl)-biphenylyl-4-methyl-amino]-1-(o-ethoxycarbonylbenzyl)-4-cyclopropylcarbonylamino-pyridin. Man kühlt ab, gießt auf Eis, macht mit Natronlauge alkalisch, arbeitet wie üblich auf und erhält 2-Cyclopropyl-4,5-dihydro-5-(o-ethoxycarbonylbenzyl)-4-oxo-3-[2'-(1H-5-tetrazolyl)biphenylyl-4-methyl]-3H-IP.

### Beispiel 4

Ein Gemisch von 1,1 g 3-p-Aminobenzyl-2-cyclopropyl-4,5-dihydro-4-oxo-5-(2-thienylmethyl)-3H-IP [erhältlich durch Reaktion von 2-Cyclopropyl-4,5-dihydro-4-oxo-5-(2-thienylmethyl)-3H-IP mit p-Nitrobenzylbromid zu 2-Cyclopropyl-4,5-dihydro-3-p-nitrobenzyl-4-oxo-5-(2-thienylmethyl)-3H-IP und anschließende Hydrierung], 0,6 g Phthalsäureanhydrid und 40 ml CHCl₃ wird 16 Stunden bei 20° gerührt. Das ausgefallene 2-Cyclopropyl-3-[4-(o-carboxybenzamido)-benzyl]-4,5-dihydro-4-oxo-5-(2-thienylmethyl)-3H-IP wird abfiltriert.

### Beispiel 5

Ein Gemisch von 3,76 g 3-p-Aminobenzyl-2-cyclopropyl-4,5-dihydro-4-oxo-5-(2-thienylmethyl)-3H-IP, 3 ml Triethylamin, 0,5 g 4-Dimethylaminopyridin und 120 ml Dichlormethan wird auf 5° gekühlt und tropfenweise mit einer Lösung von 2,88 g o-Trifluormethansulfonamidobenzoylchlorid in 20 ml Dichlormethan versetzt. Man rührt noch 16 Stunden bei 20°, dampft ein, arbeitet wie üblich auf und erhält 2-Cyclopropyl-4,5-dihydro-4-oxo-5-(2-thienyl-methyl)-3-[4-(o-trifluormethansulfonamido-benzamido)-benzyl]-3H-IP.

### Beispiel 6

Ein Gemisch von 4,72 g 2-Cyclopropyl-3-p-carboxybenzyl-4,5-dihydro-5-p-nitrophenacyl-4-oxo-3H-IP, 12 g Thionylchlorid und 35 ml CHCl₃ wird 6 Stunden gekocht und eingedampft. Das erhaltene rohe Säurechlorid wird durch mehrfaches Lösen in Toluol und Eindampfen von Thionylchlorid-Resten befreit und in 80 ml THF gelöst. Man tropft diese Lösung zu einer Lösung von 1,7 g Anthranilsäure und 0,8 g NaOH in 100 ml Wasser, rührt 24 Stunden und säuert mit Salzsäure bis pH 5 an. Nach üblicher Aufarbeitung erhält man 2-Cyclopropyl-3-[p-(2-carboxyanilino-carbonyl)-benzyl]-4,5-dihydro-5-p-nitrophenacyl-4-oxo-3H-IP.

### Beispiel 7

(a) Eine Lösung von 2,94 g 2-Cyclopropyl-3-(2'-cyan-biphenylyl-4-methyl)-4,5-dihydro-4-oxo-3H-IP (F. 183°; erhältlich durch Reaktion von 3,4-Diamino-2-chlorpyridin mit Cyclopropancarbonsäure analog Beispiel 3 (Reaktionszeit 18 Stunden) zu 2-Cyclopropyl-4,5-dihydro-4-oxo-1(oder 3)H-IP (Rf 0,27 in Ethylacetat/Methanol 8:2; FAB 176) und Umsetzung mit 4'-Brommethyl-2-cyan-biphenyl in N-Methylpyrrolidinon in Gegenwart von K₂CO₃) in 60 ml N-Methylpyrrolidinon wird unter Rühren bei 20° mit 1,25 g K-tert.-Butylat versetzt. Nach 45 Minuten Rühren wird eine Lösung von 4,6 g o-Brommethyl-benzoesäuremethylester in 25 ml DMF zugetropft. Man rührt noch 16 Stunden bei 20°, arbeitet wie üblich auf und erhält 2-Cyclopropyl-3-(2'-cyan-biphenylyl-4-methyl)-4,5-dihydro-5-(o-methoxycarbonylbenzyl)-4-oxo-3H-IP; Rf 0,41 (Petrolether/Ethylacetat 2:8); FAB 515.
   Analog erhält man die nachstehenden 2-Cyclopropyl-3-(2'-cyan-biphenylyl-4-methyl)-4,5-dihydro-4-oxo-5-R³-3H-IP:
   - mit Chloracetonitril:: -5-cyanmethyl-
   - mit 3-Brom-propionitril:: -5-(2-cyanethyl)-
   - mit 4-Brom-butyronitril:: -5-(3-cyanpropyl)-
   - mit Propargylbromid:: -5-propargyl-
   - mit Benzylbromid:: -5-benzyl-
   - mit o-Fluorbenzylbromid:: -5-(o-fluorbenzyl)-
   - mit m-Fluorbenzylbromid:: -5-(m-fluorbenzyl)-
   - mit p-Fluorbenzylbromid:: -5-(p-fluorbenzyl)-
   - mit o-Chlorbenzylbromid:: -5-(o-chlorbenzyl)-, Rf 0,48 (Petroläther/Ethylacetat 2:8)
   - mit m-Chlorbenzylbromid:: -5-(m-chlorbenzyl)-
   - mit p-Chlorbenzylbromid:: -5-(p-chlorbenzyl)-
   - mit o-Brombenzylbromid:: -5-(o-brombenzyl)-
   - mit m-Brombenzylbromid:: -5-(m-brombenzyl)-
   - mit p-Brombenyzlbromid:: -5-(p-brombenzyl)-
   - mit p-Methylbenzylbromid:: -5-(p-methylbenzyl)-
   - mit o-Trifluormethylbenzylbromid:: -5-(o-trifluormethylbenzyl)-, Rf 0,7 (Ethylacetat)
   - mit m-Trifluormethylbenzylbromid:: -5-(m-trifluormethylbenzyl)-
   - mit p-Trifluormethylbenzylbromid:: -5-(p-trifluormethylbenzyl)-
   - mit m-Methoxycarbonylbenzylbromid:: -5-(m-methoxycarbonylbenzyl)-
   - mit p-Methoxycarbonylbenzylbromid:: -5-(p-methoxycarbonylbenzyl)-
   - mit o-Ethoxycarbonylbenzylbromid:: -5-(o-ethoxycarbonylbenzyl)-
   - mit m-Ethoxycarbonylbenzylbromid:: -5-(m-ethoxycarbonylbenzyl)-
   - mit p-Ethoxycarbonylbenzylbromid:: -5-(p-ethoxycarbonylbenzyl)-
   - mit o-Cyanbenzylbromid:: -5-(o-cyanbenzyl)-
   - mit m-Cyanbenzylbromid:: -5-(m-cyanbenzyl)-
   - mit p-Cyanbenzylbromid:: -5-(p-cyanbenzyl)-
   - mit o-Nitrobenzylchlorid:: -5-(o-nitrobenzyl)-
   - mit m-Nitrobenzylchlorid:: -5-(m-nitrobenzyl)-
   - mit p-Nitrobenzylchlorid:: -5-(p-nitrobenzyl)-
   - mit o-Trifluoracetamidobenzylbromid:: -5-(o-trifluoracetamidobenzyl)-
   - mit m-Trifluoracetamidobenzylbromid:: -5-(m-trifluoracetamidobenzyl)-
   - mit p-Trifluoracetamidobenzylbromid:: -5-(p-trifluoracetamidobenzyl)-
   - mit o-Trifluormethylsulfonamidobenzylbromid:: -5-(o-trifluormethylsulfonamido-benzyl)-
   - mit m-Trifluormethylsulfonamidobenzylbromid:: -5-(m-trifluormethylsulfonamido-benzyl)-
   - mit p-Trifluormethylsulfonamidobenzylbromid:: -5-(p-trifluormethylsulfonamido-benzyl)-
   - mit 2,6-Dichlorbenzylbromid:: -5-(2,6-dichlorbenzyl)-
   - mit 2-Fluor-6-nitro-benzylbromid:: -5-(2-fluor-6-nitro-benzyl)-
   - mit 2-Chlor-6-nitro-benzylbromid:: -5-(2-chlor-6-nitro-benzyl)-
   - mit 2-Furylmethylchlorid:: -5-(2-furylmethyl)-
   - mit 2-Thienylmethylchlorid:: -5-(2-thienylmethyl)-
   - mit 5-Isoxazolylmethylbromid:: -5-(5-isoxazolylmethyl)-
   - mit 5-Methyl-3-isoxazolylmethylbromid:: -5-(5-methyl-3-isoxazolylmethyl)-
   - mit 2-Pyridylmethylchlorid:: -5-(2-pyridylmethyl)-
   - mit 4-Pyridylmethylchlorid:: -5-(4-pyridylmethyl)-
   - mit 2-(2-Furyl)-2-oxoethylbromid:: -5-(2-furoylmethyl)-
   - mit 2-(2-Thienyl)-2-oxoethylbromid:: -5-(2-thenoylmethyl)-
   - mit Brom- oder Chloraceton:: -5-(2-oxopropyl)-
   - mit Phenacylchlorid oder -bromid:: -5-phenacyl-
   - mit o-Methoxy-phenacylchlorid:: -5-o-methoxy-phenacyl-
   - mit 1-Brom-2-butanon:: -5-(2-oxobutyl)-
   - mit 1-Brom-3-methyl-2-butanon:: -5-(3-methyl-2-oxo-butyl)-
   - mit 1-Brom-3,3-dimethyl-2-butanon:: -5-(3,3-dimethyl-2-oxobutyl)-Rf 0,71 (Ethylacetat/Methanol 9:1)
   - mit o-Nitro-phenacylchlorid:: -5-o-nitro-phenacyl-
   - mit m-Nitro-phenacylchlorid:: -5-m-nitro-phenacyl-
   - mit p-Nitro-phenacylchlorid:: -5-p-nitro-phenacyl-
   - mit 1-Brom-3,3,3-trifluoraceton:: -5-(3,3,3-trifluor-2-oxopropyl)-
   - mit 1-Brom-3,3,4,4,4-pentafluor-2-butanon:: -5-(3,3,4,4,4-pentafluor-2-oxobutyl)-
   - mit 2-(3-Pyridyl)-2-oxoethylchlorid:: -5-nicotinoyl-methyl-
   - mit p-Difluormethoxyphenacylchlorid:: -5-p-difluormethoxyphenacyl-
   - mit p-Trifluormethoxyphenacylchlorid:: -5-p-trifluormethoxyphenacyl-
   - mit p-Cyanphenacylchlorid:: -5-p-cyan-phenacyl-
   - mit 2-(2-Benzofuryl)-2-oxoethylbromid:: -5-[2-(2-benzofuryl)-2-oxoethyl]-.
(b) Ein Gemisch von 3,9 g der nach (a) erhaltenen Verbindung, 5 g Trimethylzinnazid und 100 ml Toluol wird 72 Stunden gekocht und dann eingedampft. Man nimmt den Rückstand in 100 ml methanolischer HCl auf, rührt 2 Stunden bei 20° und arbeitet wie üblich auf (gesättigte NaCl-Lösung/Dichlormethan). Chromatographie (Ethylacetat/Methanol 9:1) liefert 2-Cyclopropyl-4,5-dihydro-5-(o-methoxy-carbonylbenzyl)-4-oxo-3-[2'-(1H-5-tetrazolyl)-biphenylyl-4-methyl]-3H-IP, F. 260°.
   Analog erhält man aus den unter (a) angegebenen 2'-Cyanbiphenylyl-verbindungen die nachstehenden 2-Cyclopropyl-4,5-dihydro-4-oxo-3-[2'-(1H-5-tetrazolyl)-biphenylyl-4-methyl]-5-R³-3H-IP:
   -5-propargyl-
   -5-benzyl-
   -5-(o-fluorbenzyl)-
   -5-(m-fluorbenzyl)-
   -5-(p-fluorbenzyl)-
   -5-(o-chlorbenzyl)-, F. 185°
   -5-(m-chlorbenzyl)-
   -5-(p-chlorbenzyl)-
   -5-(o-brombenzyl)-
   -5-(m-brombenzyl)-
   -5-(p-brombenzyl)-
   -5-(p-methylbenzyl)-
   -5-(o-trifluormethyl-benzyl)-
   -5-(m-trifluormethyl-benzyl)-
   -5-(p-trifluormethyl-benzyl)-
   -5-(m-methoxycarbonyl-benzyl)-
   -5-(p-methoxycarbonyl-benzyl)-
   -5-(o-ethoxycarbonyl-benzyl)-, K-Salz, F. > 300°
   -5-(m-ethoxycarbonyl-benzyl)-
   -5-(p-ethoxycarbonyl-benzyl)-
   -5-[o-(1H-5-tetrazolyl)-benzyl]-
   -5-[m-(1H-5-tetrazolyl)-benzyl]-
   -5-[p-(1H-5-tetrazolyl)-benzyl]-
   -5-(o-nitrobenzyl)-
   -5-(m-nitrobenzyl)-
   -5-(p-nitrobenzyl)-
   -5-(o-trifluoracetamido-benzyl)-
   -5-(m-trifluoracetamido-benzyl)-
   -5-(p-trifluoracetamido-benzyl)-
   -5-(o-trifluormethylsulfonamido-benzyl)-
   -5-(m-trifluormethylsulfonamido-benzyl)-
   -5-(p-trifluormethylsulfonamido-benzyl)-
   -5-(2-fluor-6-nitro-benzyl)-
   -5-(2-chlor-6-nitro-benzyl)-
   -5-(2-furylmethyl)-
   -5-(2-thienylmethyl)-
   -5-(5-isoxazolyl-methyl)-
   -5-(5-methyl-3-isoxazolyl-methyl)-
   -5-(2-pyridylmethyl)-
   -5-(3-pyridylmethyl)-
   -5-(4-pyridylmethyl)-
   -5-(2-furoylmethyl)-
   -5-(2-thenoylmethyl)-
   -5-(2-oxopropyl)-
   -5-phenacyl-
   -5-o-methoxy-phenacyl-
   -5-(2-oxobutyl)-
   -5-(3-methyl-2-oxo-butyl)-
   -5-(3,3-dimethyl-2-oxo-butyl)-, F. 153°
   -5-o-nitro-phenacyl-
   -5-m-nitro-phenacyl-
   -5-p-nitro-phenacyl-
   -5-(3,3,3-trifluor-2-oxo-propyl)-
   -5-(3,3,4,4,4-pentafluor-2-oxo-butyl)-
   -5-nicotinoyl-methyl-
   -5-p-difluormethoxy-phenacyl-
   -5-p-trifluormethoxy-phenacyl-
   -5-p-cyan-phenacyl-
   -5-[2-(2-benzofuryl)-2-oxo-ethyl]-.

### Beispiel 8

(a) Analog Beispiel 7 erhält man aus 2-Cyclopropyl-4,5-dihydro-4-oxo-3-[2'-(2-triphenylmethyl-2H-5-tetrazolyl)-biphenylyl-4-methyl]-3H-IP mit o-Brommethylbenzoesäureethylester das 2-Cyclopropyl-4,5-dihydro-5-(o-ethoxy-carbonylbenzyl)-4-oxo-3-[2'-(2-triphenylmethyl-2H-tetrazolyl)-biphenylyl-4-methyl]-3H-IP.
   Analog erhält man die nachstehenden 2-Cyclopropyl-4,5-dihyro-4-oxo-3-[2'-(2-triphenylmethyl-2H-5-tetrazolyl)-biphenylyl-4-methyl]-5-R³-3H-IP:
   -5-propargyl-
   -5-benzyl-
   -5-(o-fluorbenzyl)-
   -5-(m-fluorbenzyl)-
   -5-(p-fluorbenzyl)-
   -5-(o-chlorbenzyl)-
   -5-(m-chlorbenzyl)-
   -5-(p-chlorbenzyl)-
   -5-(o-brombenzyl)-
   -5-(m-brombenzyl)-
   -5-(p-brombenzyl)-
   -5-(p-methylbenzyl)-
   -5-(o-trifluormethyl-benzyl)-
   -5-(m-trifluormethyl-benzyl)-
   -5-(p-trifluormethyl-benzyl)-
   -5-(o-methoxycarbonyl-benzyl)-
   -5-(m-methoxycarbonyl-benzyl)-
   -5-(p-methoxycarbonyl-benzyl)-
   -5-(m-ethoxycarbonyl-benzyl)-
   -5-(p-ethoxycarbonyl-benzyl)-
   -5-[o-(1H-5-tetrazolyl)-benzyl]-
   -5-[m-(1H-5-tetrazolyl)-benzyl]-
   -5-[p-(1H-5-tetrazolyl)-benzyl]-
   -5-(o-nitrobenzyl)-
   -5-(m-nitrobenzyl)-
   -5-(p-nitrobenzyl)-
   -5-(o-trifluoracetamido-benzyl)-
   -5-(m-trifluoracetamido-benzyl)-
   -5-(p-trifluoracetamido-benzyl)-
   -5-(o-trifluormethylsulfonamido-benzyl)-
   -5-(m-trifluormethylsulfonamido-benzyl)-
   -5-(p-trifluormethylsulfonamido-benzyl)-
   -5-(2-fluor-6-nitro-benzyl)-
   -5-(2-chlor-6-nitro-benzyl)-
   -5-(2-furylmethyl)-
   -5-(2-thienylmethyl)-
   -5-(5-isoxazolyl-methyl)-
   -5-(5-methyl-3-isoxazolyl-methyl)-
   -5-(2-pyridylmethyl)-
   -5-(3-pyridylmethyl)-
   -5-(4-pyridylmethyl)-
   -5-(2-furoylmethyl)-
   -5-(2-thenoylmethyl)-
   -5-(2-oxopropyl)-
   -5-phenacyl-
   -5-o-methoxy-phenacyl-
   -5-(2-oxobutyl)-
   -5-(3-methyl-2-oxo-butyl)-
   -5-(3,3-dimethyl-2-oxo-butyl)-
   -5-o-nitro-phenacyl-
   -5-m-nitro-phenacyl-
   -5-p-nitro-phenacyl-
   -5-(3,3,3-trifluor-2-oxo-propyl)-
   -5-(3,3,4,4,4-pentafluor-2-oxo-butyl)-
   -5-nicotinoyl-methyl-
   -5-difluormethoxy-phenacyl-
   -5-trifluormethoxy-phenacyl-
   -5-p-cyan-phenacyl-
   -5-[2-(2-benzofuryl)-2-oxo-ethyl]-.
(b) Das nach (a) erhaltene Produkt (1g) wird in 60 ml 4 n HCl in Dioxan gelöst und 16 Stunden bei 20° gerührt. Man dampft ein, arbeitet wie üblich auf und erhält 2-Cyclopropyl-4,5-dihydro-5-(o-ethoxycarbonylbenzyl)-4-oxo-3-[2'-(1H-5-tetrazolyl)-biphenylyl-4-methyl]-3H-IP.
   Analog erhält man aus den unter (a) angegebenen entsprechenden 2-Triphenylmethyl-2H-5-tetrazolyl-verbindungen die in Beispiel 7b angegebenen 1H-5-Tetrazolyl-verbindungen.

### Beispiel 9

Analog Beispiel 7 erhält man aus 2-Cyclopropyl-3-(p-2-cyan-2-phenyl-vinyl-benzyl)-4,5-dihydro-4-oxo-3H-IP (erhältlich aus 2-Cyclopropyl-4,5-dihydro-4-oxo-1(oder 3)H-IP und 3-p-Brommethylphenyl-2-phenyl-acrylnitril) mit 2-Benzoyl-1-chlorethan das 5-(2-Benzoylethyl)-2-cyclopropyl-3-(p-2-cyan-2-phenyl-vinyl-benzyl)-4,5-dihydro-4-oxo-3H-IP.

### Beispiel 10

(a) Analog Beispiel 7 (a) erhält man aus 2-Cyclopentyl-3-(2'-cyanbiphenylyl-4-methyl)-4,5-dihydro-4-oxo-3H-IP (erhältlich durch Reaktion von 3,4-Diamino-2-chlorpyridin mit Cyclopentancarbonsäure analog Beispiel 3 zu 2-Cyclopentyl-4,5-dihydro-4-oxo-1(oder 3)H-IP und Umsetzung mit 4'-Brommethyl-2-cyan-biphenyl) mit o-Brommethylbenzoesäuremethylester das 2-Cyclopentyl-3-(2'-cyan-biphenylyl-4-methyl)-4,5-dihydro-5-(o-methoxycarbonylbenzyl)-4-oxo-3H-IP.
(b) Analog Beispiel 7 (b) erhält man daraus mit Trimethylzinnazid das 2-Cyclopentyl-4,5-dihydro-5-(o-methoxycarbonylbenzyl)-4-oxo-4-[2'-(1H-5-tetrazolyl)-biphenylyl-4-methyl]-3H-IP.

### Beispiel 11

Eine Lösung von 1 g 2-Cyclopropyl-4,5-dihydro-5-p-nitrophenacyl-4-oxo-3-[2'-(1H-5-tetrazolyl)-biphenylyl-4-methyl]-3H-IP in 20 ml Methanol wird an 0,3 g 5%iger Pd-Kohle bei 20° und Normaldruck bis zur Aufnahme der berechneten H₂-Menge hydriert. Man filtriert den Katalysator ab, dampft ein und erhält 5-p-Aminophenacyl-2-cyclopropyl-4,5-dihydro-4-oxo-3-[2'-(1H-5-tetrazolyl)-biphenylyl-4-methyl]-3H-IP.

Analog erhält man durch Hydrierung der in Beispiel 7b genannten entsprechenden Nitroverbindungen die nachstehenden 2-Cyclopropyl-4,5-dihydro-4-oxo-3-[2'-(1H-5-tetrazolyl)-biphenylyl-4-methyl]-3H-IP:
5-o-Amino-phenacyl-
5-m-Amino-phenacyl-.

### Beispiel 12

Eine Lösung von 2,82 g Trifluormethansulfonsäureanhydrid in 10 ml Dichlormethan wird bei -50 bis -60° zugetropft zu einer Lösung von 5,34 g 5-p-Aminophenacyl-2-cyclopropyl-4,5-dihydro-4-oxo-3-[2'-(1H-5-tetrazolyl)-biphenylyl-4-methyl]-3H-IP und 1,01 g Triethylamin in 30 ml Dichlormethan. Man läßt auf 20° erwarmen, gießt in verdünnte Essigsäure und erhält nach üblicher Aufarbeitung 2-Cyclopropyl-4,5-dihydro-4-oxo-3-[2'-(1H-5-tetrazolyl)-biphenylyl-4-methyl] -5-p-trifluormethansulfonamido-phenacyl-3H-IP.

Analog erhält man durch Acylierung der in Beispiel 11 genannten Aminoverbindungen die nachstehenden 2-Cyclopropyl-4,5-dihydro-4-oxo-3-[2'-(1H-5-tetrazolyl)-biphenylyl-4-methyl]-3H-IP:
5-o-trifluormethansulfonamidophenacyl-
5-m-trifluormethansulfonamido-phenacyl.

Die nachstehenden Beispiele betreffen pharmazeutische Zubereitungen, die Wirkstoffe der Formel I oder ihre Salze enthalten.

### Beispiel A: Tabletten und Dragees

In üblicher Weise werden Tabletten folgender Zusammensetzung gepreßt, die bei Bedarf mit einer üblichen Drageedecke auf Sucrosegrundlage überzogen werden:

| | |
|---|---|
| Wirkstoff der Formel I | 100 mg |
| Mikrokristalline Cellulose | 278,8 mg |
| Lactose | 110 mg |
| Maisstärke | 11 mg |
| Magnesiumstearat | 5 mg |
| Feinteiliges Siliciumdioxid | 0,2 mg |

### Beispiel B: Hartgelatine-Kapseln

Übliche zweiteilige Hartgelatine-Kapseln werden jeweils gefüllt mit

| | |
|---|---|
| Wirkstoff der Formel I | 100 mg |
| Lactose | 150 mg |
| Cellulose | 50 mg |
| Magnesiumstearat | 6 mg |

### Beispiel C: Weichgelatine-Kapseln

Übliche Weichgelatine-Kapseln werden mit einem Gemisch aus jeweils 50 mg Wirkstoff und 250 mg Olivenöl gefüllt.

### Beispiel D: Ampullen

Eine Lösung von 200 g Wirkstoff in 2 kg 1,2-Propandiol wird mit Wasser auf 10 l aufgefüllt und in Ampullen gefüllt, so daß jede Ampulle 20 mg Wirkstoff enthält.

## Patentansprüche

1. Imidazopyridinderivate der Formel I 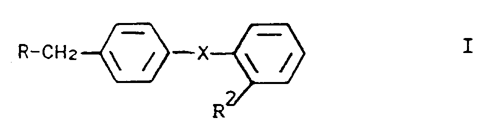 worin
R 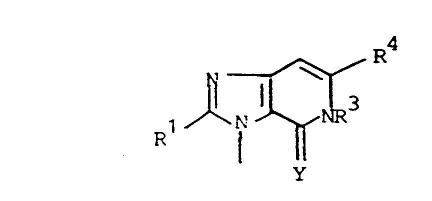
R¹ C₃-C₇-Cycloalkyl-CₙH₂ₙ- oder C₁-C₆-Alkyl, worin eine CH₂-Gruppe durch O oder S ersetzt ist,
R² H, COOR⁶, CN, NO₂, NH₂, NHCOR⁵, NHSO₂R⁵ oder 1H-5-Tetrazolyl,
R³ -CₘH₂ₘ-CN, C₂-C₆-Alkinyl, -CₘH₂ₘ-Ar, -CₘH₂ₘ-CO-R⁵, -CₘH₂ₘ-CO-Ar, -CₘH₂ₘ-Het oder -CₘH₂ₘ-CO-Het,
R⁴ H oder Hal,
R⁵ C₁-C₆-Alkyl, worin auch ein oder mehrere H-Atome durch F ersetzt sein können,
R⁶ H oder A,
X fehlt, -NH-CO-, -CO-NH-, -O-CH(COOH)-, -NH-CH(COOH)-, -NA-CH(COOH), -CH=C(COOH), -CH=C(CN) oder -CH=C(1H-5-tetrazolyl)-,
Y O oder S,
A Alkyl mit 1-6 C-Atomen,
Ar eine unsubstituierte oder eine durch Hal, R⁵, OH, OR⁵, COOR⁶, CN, NO₂, NH₂, NHA, N(A)₂, NHCOR⁵, NHCOOA, NHSO₂R⁵ und/oder 1H-5-Tetrazolyl monosubstituierte oder disubstituierte Phenylgruppe,
Het einen fünf- oder sechsgliedrigen heteroaromatischen Rest mit 1 bis 4 N-, O- und/oder S-Atomen, der auch mit einem Benzol- oder Pyridinring kondensiert sein kann,
Hal F, Cl, Br oder I,
m 1, 2, 3, 4, 5 oder 6 und
n 0, 1, 2, 3, 4, 5 oder 6 bedeuten,
sowie ihre Salze.

2. a) 2-Cyclopropyl-4,5-dihydro-5-(o-methoxycarbonylbenzyl)-4-oxo-3-[2'-(1H-5-tetrazolyl)-biphenylyl-4-methyl]-3H-imidazo[4,5-c]pyridin;
b) 2-Cyclopropyl-5-(o-ethoxycarbonylbenzyl)-4,5-dihydro-4-oxo-3-[2'-(1H-5-tetrazolyl)-biphenylyl-4-methyl]-3H-imidazo[4,5-c]pyridin;
c) 2-Cyclopentyl-4,5-dihydro-5-(o-methoxycarbonylbenzyl)-4-oxo-3-[2'-(1H-5-tetrazolyl)-biphenylyl-4-methyl]-3H-imidazo[4,5-c]pyridin;
d) 2-Cyclopropyl-5-(o-chlorbenzyl)-4,5-dihydro-4-oxo-3-[2'-(1H-5-tetrazolyl)-biphenylyl-4-methyl]-3H-imidazo[4,5-c]pyridin.

3. Verfahren zur Herstellung von Imidazopyridinen der Formel I nach Anspruch 1 sowie ihrer Salze, dadurch gekennzeichnet, daß man
(a) eine Verbindung der Formel II 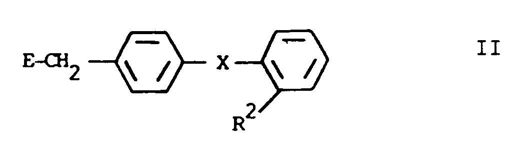 worin
E Cl, Br, I oder eine freie oder reaktionsfähig funktionell abgewandelte OH-Gruppe bedeutet und
R² und X die in Anspruch 1 angegebene Bedeutung haben,
mit einer Verbindung der Formel III
H-R III
worin
R die in Anspruch 1 angegebene Bedeutung hat,
umsetzt
oder
(b) eine Verbindung der Formel IV 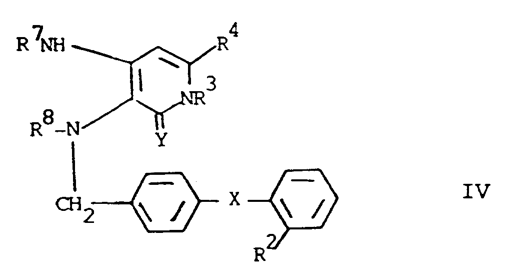 worin
R⁷ R¹-CO oder H und
R⁸ H (falls R⁷ R¹-CO ist) oder R¹-CO (falls R⁷ H ist)
bedeuten,
und
R¹, R², R³, R⁴, X und Y die in Anspruch 1 angegebenen Bedeutungen haben,
mit einem cyclisierenden Mittel behandelt,
oder
(c) zur Herstellung einer Verbindung der Formel I,
worin
X -NH-CO- oder -CO-NH- bedeutet,
eine Verbindung der Formel V 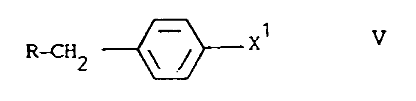 worin
X¹ NH₂ oder COOH bedeutet und
R die in Anspruch 1 angegebene Bedeutung hat
oder ein reaktionsfähiges Derivat dieser Verbindung mit einer Verbindung der Formel VI 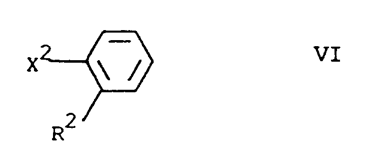 worin
X² COOH (falls X¹ NH₂ ist) oder NH₂ (falls X¹ COOH ist) bedeutet und
R² die in Anspruch 1 angegebene Bedeutung hat,
oder mit einem reaktionsfähigen Derivat dieser Verbindung umsetzt,
oder
(d) eine Verbindung der Formel I aus einem ihrer funktionellen Derivate durch Behandeln mit einem solvolysierenden oder hydrogenolysierenden Mittel in Freiheit setzt, oder
(e) eine sonst der Formel I entsprechende Verbindung, die jedoch an Stelle des Restes R³ ein H-Atom enthält, mit einer Verbindung der Formel E-R³, worin E und R³ die oben angegebenen Bedeutungen haben, behandelt,
und/oder daß man in einer Verbindung der Formel I einen oder mehrere Rest(e) R und/oder R² in einen oder mehrere andere Reste R und/oder R² umwandelt und/oder eine Base oder Säure der Formel I in eines ihrer Salze umwandelt.

4. Verfahren zur Herstellung pharmazeutischer Zubereitungen, dadurch gekennzeichnet, daß man eine Verbindung der Formel I nach Anspruch 1 und/oder eines ihrer physiologisch unbedenklichen Säureadditionssalze zusammen mit mindestens einem festen, flüssigen oder halbflüssigen Träger- oder Hilfsstoff in eine geeignete Dosierungsform bringt.

5. Pharmazeutische Zubereitung, gekennzeichnet durch einen Gehalt an mindestens einer Verbindung der Formel I nach Anspruch 1 und/oder einem ihrer physiologisch unbedenklichen Säureadditionssalze.

6. Verbindung der Formel I nach Anspruch 1 und ihre physiologisch unbedenklichen Säureadditionssalze zur Bekämpfung von Krankheiten.

7. Verwendung von Verbindungen der Formel I nach Anspruch 1 und/oder ihrer physiologisch unbedenklichen Säureadditionssalze zur Herstellung eines Arzneimittels.

8. Verwendung von Verbindungen der Formel I nach Anspruch 1 und/oder ihrer physiologisch unbedenklichen Säureadditionssalze bei der Bekämpfung von Krankheiten.
